# EUROPÄISCHE PATENTANMELDUNG

(11) **EP 1 537 865 A1**
(43) Veröffentlichungstag der Anmeldung: **08.06.2005**
(21) Anmeldenummer: 04001650.3
(22) Anmeldetag: 27.01.2004
(51) Int. Cl.: A61K 31/198, A61K 38/06, A61K 31/10, A61K 31/185, A61K 31/19, A61P 3/10, A61K 31/194, A61K 38/48

(54) **Verwendung von schwefelhaltigen Verbindungen und proteolytischen Enzymen zur Prophylaxe und Therapie des UDP-Glucuronosyl-transferase 1-defizitären Diabetes mellitus Typ II**

(30) Priorität: 03.12.2003 EP 03027678
(71) Anmelder: Liebel, Franz-Peter, Dr., 72663 Grossbettlingen (DE)
(72) Erfinder: Liebel, Franz-Peter, Dr., 72663 Grossbettlingen (DE)

(57) **Zusammenfassung**

Die Erfindung wird zur Prophylaxe und Therapie des hier so benannten L-Diabetes und damit assoziierter Erkrankungen verwendet, wobei der L-Diabetes eine Teilmenge des Diabetes mellitus darstellt, der verursacht ist durch überhöhte Konzentrationen ausscheidungspflichtiger Toxine, die wiederum die Folge insbesondere einer unzureichenden Glukuronidierung durch die UDP-Glucuronosyltransferase 1 sowie einer unphysiologisch hohen Bildung von Giftstoffen im Dickdarm sind.
Es besteht also ein UDP-Glucuronosyltransferase 1-Defizit, so daß zum Ausgleich vermehrt schwefelverbrauchende Konjugationen herangezogen werden müssen mit dem Resultat einer Überbeanspruchung des Sulfat- und Glutathionpools.
Konsequenterweise wird zur Prophylaxe und Therapie des L-Diabetes neben anderen Stoffen, wie Glutathion, Taurin, L-Cystin und L-Methionin zuvorderst N-Acetylcystein eingesetzt. Die bei unzureichender Proteinverdauung vermehrt anfallenden Amine, Mercaptane sowie Phenol- und Indolkörper, die im wesentlichen Glukuronsäure- oder Sulfat-Entgiftungsmechanismen unterliegen, werden durch den Einsatz von Pepsin, Citronensäure und Lactulose verringert.

## Beschreibung

### A) Verwendbarkeit der Erfindung

Die Erfindung wird verwendet zur Prophylaxe und Therapie des hier so benannten L-Diabetes und der damit assoziierten Erkrankungen, wobei der L-Diabetes eine Teilmenge des Diabetes mellitus darstellt, der verursacht ist durch überhöhte Konzentrationen ausscheidungspflichtiger Toxine, die wiederum die Folge insbesondere einer unzureichenden Glukuronidierung durch die UDP-Glucuronosyltransferase1 (UGT1) sowie einer unphysiologisch hohen Bildung von Giftstoffen im Dickdarm sind.

Da bei 8 % der Kaukasier UGT1A1 und damit verknüpft fast immer auch UGT1A6 sowie UGT1A7 jeweils ohne Wildtyp-Allel vorliegen, ist bei diesem Teil der Bevölkerung mit einem hohen Entgiftungsdefizit und einem nicht unerheblichen Anteil an L-Diabetes zu rechnen.

Die verwendeten schwefeltragenden Verbindungen erhöhen wirksam die Möglichkeiten zur Sulfatierung, Methylierung, Glutathionkonjugation, Taurinkonjugation und Metallothionein-Synthese, und man erreicht so die Entgiftung von endogenen und exogenen Toxinen, die über direkte Zellschädigung, über Proteinkopplung oder über Ah-Rezeptoren den L-Diabetes in Gang setzen.

Die verbesserte Proteolyse mindert die Konzentrationen an Aminen, Mercaptanen, Phenol- und Indolkörpern, die im Hinblick auf die Entgiftungsmechanismen mit den exogenen Toxinen konkurrieren. Diese Giftminderung ist bei mangelhafter Glukuronidierung und überbeanspruchtem Sulfatpool von wesentlicher Bedeutung.

### B) Stand der Technik und Vorteile des Verfahrens

Die Genese des Diabetes mellitus Typ II ist derzeit allenfalls in Teilschritten geklärt. Hier wird postuliert, daß die bei unzureichender Proteinverdauung entstehenden Gifte sowie eine - mit Sicherheit unvollständig aufgelistete - Auswahl von exogenen Toxinen infolge des bestehenden Defektes der physiologischen Entgiftungsmechanismen in der Lage sind, durch die Schädigung der Strukturen oder der metabolischen Abläufe von Zielzellen sowie durch die Anheftung an Proteine oder durch die Einflußnahme über Arylhydrocarbon-Rezeptoren (Ah-Rezeptoren) an der Entstehung des L-Diabetes entscheidend mitzuwirken.

Als Gifte kommen zuvorderst Amine in Betracht, die bei ungenügender Proteinverdauung im Dickdarm durch Decarboxylierung von Aminosäuren entstehen. Ferner werden durch den bakteriellen Abbau von nicht resorbiertem Phenylalanin, Tyrosin, Tryptophan und Methionin nicht unerhebliche Mengen an Phenolkörpem bzw. Indolkörpem bzw. Mercaptanen gebildet. Diese mehr oder weniger toxischen Substanzen werden zum Teil resorbiert und gelangen über die Pfortader zur Leber, wo sie u.a. durch Kopplung an Sulfat oder Glukuronsäure für die Nieren ausscheidungsfähig gemacht werden müssen. Infolge der nur eingeschränkt möglichen Glukuronidierung wird dafür ein wesentlicher Teil des Schwefelpools verbraucht.

Eine Störung der Pepsinwirkung und demzufolge eine ungenügende Proteinverdauung mit vermehrt benötigten Sulfat-, Glutathion- und Glukuronsäure-Entgiftungen kann sich leicht einstellen. Die Vermeidung eines solchen Mißstandes mit Hilfe einer Zufuhr von Pepsin- und Citronensäure sowie von Lactulose ist deshalb von Bedeutung.

Exogene Toxine sind unter anderem die in der nachfolgenden tabellarischen Auflistung enthaltenen Stoffe. Für die Positionen 1) bis 9) sind Schwefelverbindungen, für die Positionen 10) bis 13) ist Glukuronsäure das hauptsächliche Mittel zur Aufhebung der Giftwirkung.

| **Namensgebung**: Abkürzende Benennung der u.a. durch ein UDP-Glucuronosyltransferase 1-Defizit und eine Proteinfäulnis im Colon charakterisierten Diabetesform als **L-Diabetes** nach dem Patentanmelder Liebel, Franz-Peter, Dr. | | |
|---|---|---|
| Toxin | Zufuhr durch: | Hauptsächliche Entgiftung: |
| 1) Acrylamid | Brot, Gebäck, Kaffee, Kakao, Bratkartoffel | Glutathionkopplung |
| 2) Polychlorierte Biphenyle (PCB) | Milch, Fleisch, Fisch | Glutathionkopplung |
| 3) Polycyclische aromatische Kohlenwasserstoffe (PAK) | Pyrolyseprodukte, Kaffee, Milch, Blattgemüse | Glutathionkopplung, Glukuronidierung, Sulfatierung |
| 4) Arsen | Inhalative Aufnahme | Glutathion antagonisiert |
| 5) Quecksilber | Frischwasser- und Seefische, Meeresfrüchte | Glutathion bindet Hg-Verbindungen |
| 6) Blei | Obst, Gemüse, Atemluf | Cystein |
| 7) Patulin | Mehlprodukte, Fleisch, braunfaule Äpfel | Hohe Affinität zu SH-Gruppen |
| 8) Penicillinsäure | Lebensmittel, Tierfutter | Führt zu Glutathion-Depletion |
| 9) Trichothecene | Getreide, vor allem Weizen, Tierfutter | Hohe Affinität zu SH-Gruppen |
| 10) Dibenzodioxine, Dibenzofurane | Milch, Fleisch, Eier | Nach Hydroxylierung entgiftet durch Glukuronidierung |
| 11) Pentachlorphenol (PCP) | Zufuhr durch verunreinigte Lebensmittel | Glukuronidierung |
| 12) Chlorogensäure, Kaffeesäure, Protocatechusäure, | Möhren, Sellerie, Kaffee, Schwarztee, Kakao, Wein, Kartoffel, Kopfsalat, Endivie, Kohl, Äpfel, Beeren | Nach der Metabolisierung zu einfachen Phenolen erfolgt Glukuronidierung |
| 13) Cumarine | Getreide, Hülsenfrüchte, Nachtschatten, Fenchel, Citrus, Aprikosen, Erdbeeren, Kirschen, Möhreu | Nach der Hydroxylierung erfolgen Glukuronidierung und andere Konjugationen |

Von Vorteil ist es nun, wenn man für die Toxine, die bevorzugt glukuronidiert werden, durch Sulfatierung, Methylierung, Glutathionkopplung oder Taurinkonjugation doch noch die Eliminierung erreicht. Dies wird gefördert durch die verabreichten schwefelhaltigen Verbindungen und die damit verbundene erhöhte Bereitstellung von Sulfat, Methylgruppen, Glutathion und Taurin.

Die außerdem noch existierenden Konjugationsmechanismen, nämlich Acetylierung und Konjugation mit Aminosäuren, kommen für eine mögliche Beeinflussung nicht in Betracht, da Substratdefizite an Acetat bzw. Aminosäuren nicht entstehen.

### C) Darstellung der Lösung und Beispiel

Das aus dem Methioninstoffwechsel gewonnene oder durch N-Acetylcystein zugeführte L-Cystein wird wie folgt zu Sulfat und Glutathion umgesetzt:

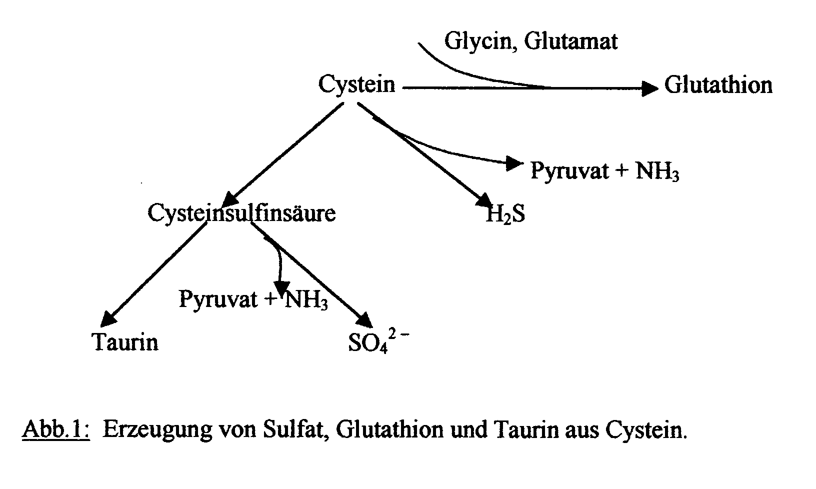

Am Beispiel der Phenolcarbonsäuren und ihrer Ester werden die Auswirkungen einer mangelhaften Glukuronidierung gezeigt.

Als Phenolcarbonsäuren bezeichnet man Hydroxyzimtsäuren (z.B. o-Cumarsäure, p-Cumarsäure, Kaffeesäure) und Hydroxybenzoesäuren (z.B. Salicylsäure, p-Hydroxybenzoesäure, Protocatechusäure). Ein Ester der Kaffeesäure ist die in vielen Pflanzen enthaltene Chlorogensäure.

Aus den Hydroxyzimtsäuren können durch Abbau der Seitenkette über den Weg der β-Oxidation Hydroxybenzoesäuren entstehen, die wiederum durch Decarboxylierung einfache Phenole ergeben.

Für die metabolische Aktivierung des Phenols gilt: Phenol→ Hydrochinon → p-Benzochinon. Das p-Benzochinon ist Startsubstanz des folgenden Schädigungszyklus:

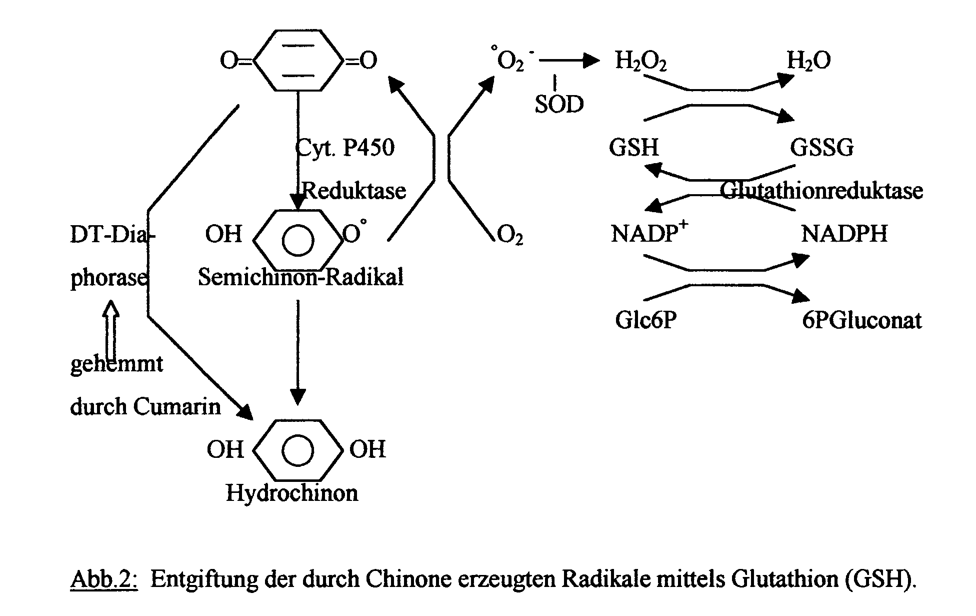

Aus Abb. 2 sind bei mangelnder Phenol-Glukuronidierung infolge eines UGT1-Defekts drei Schädigungsmechanismen zu entnehmen:
1.
   Die durch den UGT1-Defekt bewirkte unzureichende Glukuronidierung des Phenols muß ersetzt werden durch andere Entgiftungsmechanismen, z.B. durch eine Sulfatierung oder Glutathionkopplung, bei entsprechendem Verbrauch.
2.
   Das nicht oder nur verzögert eliminierte Phenol erzeugt Sauerstoffradikale, aus denen mit Hilfe der Superoxiddismutase H₂O₂ entsteht, dessen Metabilisierung zu H₂O Glutathion beansprucht und so den Glutathionpool weiter mindert.
3.
   Cumarine, die infolge der generell verminderten Glukuronidierung erhöht vorliegen, und die durch Hemmung der DT-Diaphorase die unmittelbare Bildung von Hydrochinon aus p-Benzochinon behindern, erhöhen die Belastung mit Semichinon-Radikalen; die Eliminierung der Radikale steigert den Glutathionverbrauch weiter.

Die Bedeutung und die Wirkung der in den Patentansprüchen 1 - 3 aufgelisteten Substanzen wird wie folgt zusammengefaßt:

### L-Methionin

Die Aminosäure Methionin ist Ausgangssubstanz eines zu Cystein führenden Stoffwechselwegs, wobei gleichzeitig über S-Adenosylmethionin die Methylgruppen für Methylierungen bereitgestellt werden. Vorsicht ist jedoch geboten, da aus nichtresorbiertem Methionin durch bakteriellen Abbau im Colon die extrem toxischen Mercaptane entstehen.

### N-Acetylcystein

N-Acetylcystein erhöht nach Deacetylierung den Pool an Cystein, das die Versorgung mit Glutathion, Sulfat und Taurin sicherstellt. (Siehe Abb. 1.)

### Glutathion und Taurin

Die Zufuhr von Glutathion, das als Tripeptid u.U. ohne Proteolyse die Darmwand passieren kann, sowie von Taurin ermöglicht es, daß Cystein eingespart und der leicht zu erschöpfende Sulfatpool begünstigt werden kann. Eventuell erreichte hohe Taurin- und Glutathionspiegel bringen einen weiteren Vorteil, da Taurin und Glutathion - neben ihrer Befähigung zur Konjugatbildung - auch direkt mit Radikalen wie ^{°}O₂ ⁻ reagieren.

### L-Cystin

Außerhalb der Zelle existiert Cystein nur in oxidierter Form als Disulfid Cystin, aus dem durch hydrierende Spaltung der Disulfidbrücke mittels Cystinreduktase Cystein gewonnen wird.

Die intestinale Resorption von Cystin konkurriert mit der Resorption von Lysin, Arginin und Omithin, da die vier Aminosäuren infolge ihrer strukturellen Ähnlichkeit ein gemeinsames Transportsystem benutzen. Die Einnahme von L-Cystin zusammen mit einer proteinreichen Mahlzeit ist deshalb in zweierlei Hinsicht unvorteilhaft. Zum einen wird die Resorption des benötigten Cystins behindert, zum andern gelangen die u.U. nicht resobierten Aminosäuren Lysin, Arginin und Ornithin in den Dickdarm und werden dort bakteriell zu Aminen decarboxyliert.

### Pepsin und Citronensäure

Das im Magensaft enthaltene Pepsin spaltet im Inneren der Proteinmoleküle, vor allem vor und nach aromatischen Aminosäuren. Pepsin ist jedoch das einzige Verdauungsenzym, das vor den aromatischen Aminosäuren zu spalten vermag.

Das pH-Optimum für Pepsin beträgt 1 - 2. Citronensäure wird zugeführt, damit dieser pH-Wert auch erreicht und ausreichend lange aufrecht erhalten wird. Hierzu ist es zweckmäßig,
- bei proteinreichen Mahlzeiten den pH-Wert des Magens nicht durch Getränke zu erhöhen,
- alkalisierende Bestandteile der Mahlzeit zu minimieren oder zeitlich nach Möglichkeit von den Proteinträgem zu trennen,
- Proteine in denaturierter Form und nur in kleineren Mengen je Mahlzeit einzunehmen und
- bei vorhandener oder zu erwartender Erhöhung der Sympathikusaktivität, z.B. infolge körperlicher oder psychischer Belastung, keine Proteinmahlzeiten einzunehmen, da die intestinale Resorptionsleistung zurückgeht.

Es ist zu beachten, daß eine ungesunde Lebensweise - charakterisiert durch die Mißachtung der oben aufgeführten vier Ernährungsregeln - leicht eine nicht-optimale Wirkung des Pepsins provoziert.

### Proteolytische Enzyme des Pankreas

Im Gegensatz zum Pepsin des Magensekrets besteht bei den proteolytischen Enzymen des Pankreas keine Gefahr einer Wirkungshemmung durch verfehlte pH-Werte. Die Substitution von Pankreasenzymen - unter Berücksichtigung der reichlichen Pankreassekretion von bis zu 3000 ml pro Tag und der reichlichen Enzymausstattung des Sekrets - wird deshalb vornehmlich bei einer diagnostizierten exkretorischen Pankreasinsuffizienz von Nutzen sein.

### Lactulose

Eine durch Lactulose bewirkte Ansäuerung des Dickdarms bewirkt eine Begünstigung von Bifidumbakterien, die weder Phenol noch Ammoniak bilden. Lactulose hat, neben einer wesentlichen Förderung der Ammoniakausscheidung, noch einen durchaus erwünschten laxierenden Effekt.

Es bleibt zu klären, welcher pathogenetischer Mechanismus dazu führt, daß eine verminderte Glukuronidierung einen L-Diabetes und weitere häufig damit verknüpfte Krankheitsbilder erzeugen kann. Solche begleitenden, aber auch isoliert auftretenden Erkrankungen, sie sind im Patentanspruch 1 im einzelnen genannt, treten deshalb zusammen mit dem L-Diabetes auf, da sie ganz ohne Zweifel gemeinsame Ursachen besitzen. Aus den vorangegangenen Aussagen und Zusammenhängen ergibt sich als Hypothese die kompakt gefaßte Ablaufskizze der Abb.3.

Hierbei ist zu beachten, daß Bilirubin ein sehr wirksamer Radikalenfänger ist, und daß deshalb ein aktivitätsgemindertes UGT1A1, das Bilirubin in erhöhter Konzentration im Serum beläßt, eine (in Abb.3 eingetragene) mildernde Wirkung hat im Hinblick auf die Folgen der übrigen leistungsschwachen UGT1-Isoenzyme UGT1A6 und UGT1A7, die für Radikale verantwortlich zu machen sind im Gefolge der ungenügenden Beseitigung phenolischer Verbindungen.

Diese Begünstigung wird wieder aufgewogen durch die Bindung des Bilirubins an das in Membranen enthaltene Lecithin und seine (in Abb.3 eingetragene) Wirkung als Detergens.

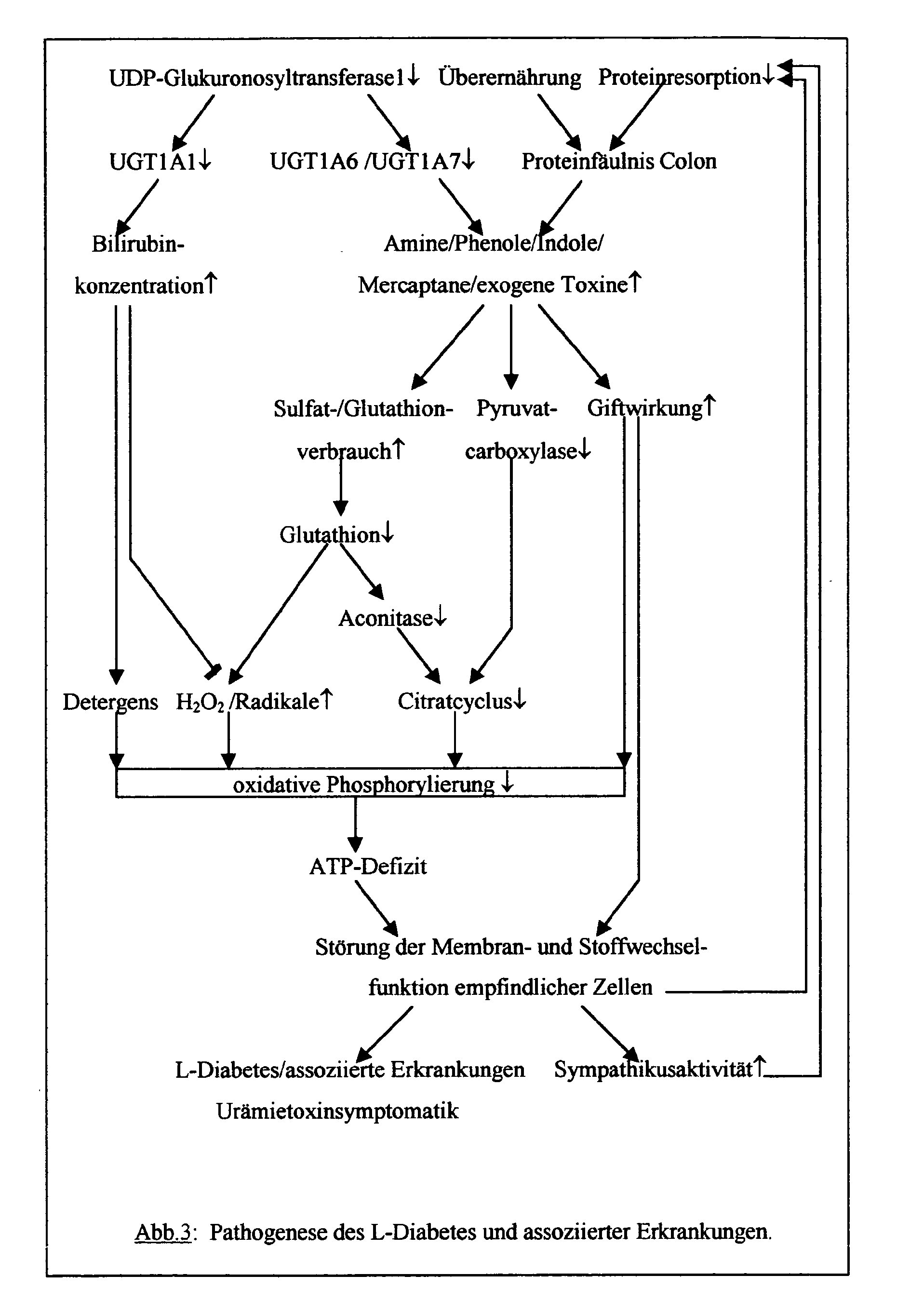

## Patentansprüche

1. Die Erfindung ist dadurch charakterisiert, daß sie zur Prophylaxe und Therapie des hier so benannten L-Diabetes und damit assoziierter Erkrankungen verwendet wird, wobei der L-Diabetes eine Teilmenge des Diabetes mellitus darstellt, der verursacht ist durch überhöhte Konzentrationen ausscheidungspflichtiger Toxine, die wiederum die Folge insbesondere einer unzureichenden Glukuronidierung durch die UDP-Glucuronosyltransferase 1 sowie einer unphysiologisch hohen Bildung von Giftstoffen im Dickdarm sind, und daß zur Prophylaxe und Therapie dieses L-Diabetes möglichst frühzeitig, insbesondere wenn
- Bilirubin und/oder
- Glutathionreduktase zu hoch sowie
- Glutathion zu niedrig
getestet werden, oder wenn
- Urämietoxinsymptome oder Leberüberlastsymptome
festzustellen sind, spätestens aber bei diagnostiziertem Diabetes mellitus Typ II, eine molekulargenetische Überprüfung der UDP-Glucuronosyltransferase 1 (UGT1) erfolgt, wobei nach derzeitigen Erkenntnissen etwa ein Dutzend Isoenzyme zur Überprüfung anstehen, wobei aber insbesondere das Vorliegen der Allele
- UGT1A1*28,
- UGT1A6*2,
- UGT1A6 R184S,
- UGT1A7*2 und
- UGT1A7*3
getestet werden soll, und daß dann bei einem festgestellten UGT1-Defizit, das über einen UGT1A1-Mangel hinausgeht, zur Prophylaxe und Therapie des langfristig sich daraus entwickelnden L-Diabetes sowie zur Prophylaxe und Therapie der häufig damit assoziierten, jedoch auch isoliert auftretenden Erkrankungen, insbesondere der
- peripheren Neuropathie,
- distalen Muskelatrophie,
- männlichen Sexualfunktionsstörungen,
- extremen Muskelermüdbarkeit,
- Funktionsstörungen der Ganglienzellen des Gehirns,
- Arthritis und der
- Urämietoxinsymptomatik,
die schwefelhaltigen Verbindungen
- N-Acetylcystein
- Glutathion,
- Taurin,
- L-Cystin und
- L-Methionin,
sowie vor einer proteinreichen Mahlzeit zur Minderung der bei ungenügender Proteinverdauung im Dickdarm entstehenden Toxine zusätzlich
- Pepsin zusammen mit Citronensäure und
zur Begünstigung von im Dickdarm angesiedelten Bifidumbakterien
- Lactulose
zugeführt werden, wobei als eine die schwefelhaltigen Verbindungen betreffende und in weiten Grenzen variable Empfehlung dienen mag, zweimal täglich 200 mg N-Acetylcystein, zu methioninarmen Proteinmahlzeiten 250 mg L-Methionin sowie ergänzend eine Auswahl aus jeweils 100 mg Glutathion, Taurin und L-Cystin einzunehmen, und dann eine solche Startversion unter Beachtung der Methionin- und Cystingegenanzeigen so zu variieren, daß Glutathion und die Glutathionreduktase möglichst innerhalb der Norm gemessen werden.

2. Verwendung der Stoffe gemäß Anspruch 1, wenn die dort aufgelisteten Substanzen durch die proteolytischen Enzyme
- Trypsin,
- Chymotrypsin,
- Carboxypeptidase A und B,
- Papain und
- Bromelaine
ergänzt werden, und wenn man zur Senkung des Magen-pH und damit zur Optimierung der Pepsinwirkung anstelle oder zusammen mit der Citronensäure
- verdünnte HCl-Lösung oder
- Essig
verwendet.

3. Verwendung der Stoffe gemäß Anspruch 1 und 2, wenn diese Stoffe in variierender Anzahl und Menge zu Kombinationspräparaten zusammengestellt werden, oder wenn man diese Stoffe einzeln oder in Kombinationen und in variierenden Mengen Getränken bzw. Nahrungsmitteln beimischt.
